(19) Europäisches Patentamt / European Patent Office / Office européen des brevets

(11) **EP 4 029 467 A1**

(12) **EUROPEAN PATENT APPLICATION**
published in accordance with Art. 153(4) EPC

(43) Date of publication:
**20.07.2022 Bulletin 2022/29**

(21) Application number: **20863119.2**

(22) Date of filing: **08.09.2020**

(51) International Patent Classification (IPC):
**A61B 34/37** (2016.01)   **A61B 34/30** (2016.01)

(86) International application number:
**PCT/CN2020/114115**

(87) International publication number:
**WO 2021/047522 (18.03.2021 Gazette 2021/11)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **10.09.2019 CN 201910854118**

(71) Applicant: **Shenzhen Jingfeng Medical Technology Co., Ltd.**
**Shenzhen, Guangdong 518000 (CN)**

(72) Inventors:
• **GAO, Yuanqian**
  **Shenzhen, Guangdong 518000 (CN)**
• **YE, Guoqiang**
  **Shenzhen, Guangdong 518000 (CN)**

(74) Representative: **Zaboliene, Reda**
**Metida**
**Business center Vertas**
**Gyneju str. 16**
**01109 Vilnius (LT)**

(54) **SURGICAL ROBOT, AND CONTROL METHOD AND CONTROL DEVICE FOR DISTAL INSTRUMENT THEREOF**

(57)    A control method and device of a surgical robot and an end instrument (34) are provided. The control device is configured for acquiring first target position and/or pose information of a remote end of a mechanical arm (21) at a first coordinate system and current position and/or pose information of each controlled end instrument (34) (S1); converting the current position and/or pose information of each controlled end instrument (34) at the first coordinate system to second target position and/or pose information of the controlled end instrument at a second coordinate system (S2); controlling joint assemblies (210-214) of the mechanical arm (21) to linkedly move according to the first target position and/or pose information, and controlling the joint assemblies of the operation arms (31) to linkedly move according to the second target position and/or pose information to allow the controlled end instrument (34) to maintain current position and/or pose (S3). The surgical robot can adjust the remote end of the mechanical arm (21) while remaining the current pose of the controlled end instrument (34) unchanged.

FIG. 4

## Description

The present disclosure claims a priority of a Chinese patent application No. 201910854118.3, filed on September 10, 2019 in China, titled "surgical robot, and control method and control device for end instrument thereof". All disclosures of the Chinese patent application may be quoted by the present disclosure.

FIELD

The subject matter herein generally relates to surgical systems, in particular to a surgical robot, and a control method and a control device for end instrument of the surgical robot.

BACKGROUND

Minimally invasive surgery refers to a surgical method of performing a procedure in a human body cavity using modern medical instruments such as laparoscopes, thoracoscopes, and so on. Compared with traditional surgery modes, minimally invasive surgery has advantages of being in little trauma, little pain, fast recovery, and the like.

With advances in science and technology, minimally invasive surgical technologies are increasingly mature and widely used. Minimally invasive surgical robots usually include a master console and a slave operating device, the master console includes a handle, a doctor sends a control command to the slave operating device by operating the handle, the slave operating device includes a mechanical arm and a plurality of operating arms arranged on a remote end of the mechanical arm. The operating arm has an end instrument positioned at a remote end thereof, which moves with the handle to achieve remote surgical operation.

During operation, adjustment of the remote end of the mechanical arm results movements of the operating arms with the end instruments, and in particular, with these end instruments, a change of the position and/or pose of the end instrument easily causes discomfort or accident for trauma.

SUMMARY

Based on this, it is necessary to provide a control method for controlling end instruments of a surgical robot during a surgical process, and a control device, a computer readable storage medium, and the surgical robot based on the control method.

In one aspect, a control method for controlling an end instrument of a surgical robot is provided. The surgical robot includes a mechanical arm, the mechanical arm has one or more operating arms mounted on a remote end thereof, and the one or more operating arms each has an end instrument being a controlled end instrument, the control method includes the following steps: acquiring first target position and/or pose information of the remote end of the mechanical arm at a first coordinate system and current position and/or pose information of each of the controlled end instrument at the first coordinate system, the first coordinate system being a base coordinate of the mechanical arm; in a condition of the remote end of the mechanical arm reaching a target position and/or pose corresponding to the first target position and/or pose information, converting the current position and/or pose information of each of the controlled end instrument at the first coordinate system to second target position and/or pose information of a corresponding controlled end instrument at a second coordinate system, the second coordinate system being a tool coordinate system of the mechanical arm, controlling joint assemblies of the mechanical arm to linkedly move according to the first target position and/or pose information to allow the remote end of the mechanical arm to reach the target position and/or pose corresponding to the first target position and/or pose information, and controlling joint assemblies of the one or more operating arms to linkedly move according to the second target position and/or pose information to allow the controlled end instrument to maintain a current position and/or pose unchanged.

Before the step of acquiring the current position and/or pose information of the controlled end instrument at the first coordinate system, the control method further includes acquiring a selection instruction input by a user, and selecting one or more end instruments according to the selection instruction to be the controlled end instruments.

After the step of converting the current position and/or pose information of each of the controlled end instrument at the first coordinate system to be the second target position and/or pose information of the corresponding controlled end instrument at the second coordinate system, the control method further includes determining if each second target position and/or pose information is effective; and if each second target position and/or pose information is effective, performing the step of controlling the joint assemblies of the mechanical arm to linkedly move according to the first target position and/or pose information to allow the remote end of the mechanical arm to reach the target position and/or pose corresponding to the first target position and/or pose information, and controlling the joint assemblies each of the one or more operating arms to linkedly move according to the corresponding second target position and/or pose information to allow the controlled end instrument to maintain the current position and/or pose unchanged.

In the step of determining if each second target position and/or pose information is effective, the control method further includes determining if the second target position and/or pose information is effective, and acquiring a corresponding determined result; and determining if each second target position and/or pose information is effective according to the determined result.

In the step of determining if each second position and/or pose information is effective, the control method further includes analyzing the second target position and/or pose information to obtain target motion state parameters of each joint assembly in each of the one or more operating arms; comparing the target motion state parameters of each joint assembly in each of the one or more operation arms with motion state thresholds of each joint assembly of each of the one or more operating arms; if the target motion state parameters of each joint assembly in each of the one or more operation arms are not greater than the motion state thresholds of each joint assembly of each of the one or more operating arm, determining that the second target position and/or pose information is effective; otherwise, determining that the corresponding second target position and/or pose information is ineffective.

The target motion state parameters include position parameters, speed parameters, and accelerated speed parameters, and the motion state thresholds include a position parameter threshold, a speed parameter threshold, and an accelerated speed parameter threshold.

In the step of acquiring the first target position and/or pose information of the remote end of the mechanical arm at the first coordinate system, the control method further includes acquiring configuration information of task degrees of freedom of the remote end of the mechanical arm; and according to the configuration information of task degrees of freedom of the remote end of the mechanical arm, acquiring the first target position and/or pose information of the remote end of the mechanical arm at the first coordinate system and associated with the corresponding task degrees of freedom.

The configuration information of the task degrees of freedom of the remote end of the mechanical arm is associated with position information and/or pose information.

In the step of acquiring the current position and/or pose information of the controlled end instrument at the first coordinate system, the control method further includes acquiring configuration information of task degrees of freedom of each controlled end instrument; and according to the configuration information of task degrees of freedom of the controlled end instrument, obtaining the current position and/or pose information of the remote end of the mechanical arm at the first coordinate system and associated with the corresponding task degrees of freedom.

There are more than two controlled end instruments, the controlled end instruments have the same configuration information of the task degrees of freedom, and the configuration information of the task degrees of freedom is associated with a position freedom degree and/or pose freedom degree.

In the step of acquiring the first target position and/or pose information of the remote end of the mechanical arm at the first coordinate system, the control method further includes acquiring a six-dimensional torque vector of a force applied to the mechanical arm to pull the remote end of the mechanical arm to move; analyzing the six-dimensional torque vector of the force to obtain increment position and/or pose information of the remote end of the mechanical arm at the first coordinate system; acquiring the current position and/or pose information of the remote end of the mechanical arm at the first coordinate system; calculating the first target position and/or pose information according to the increment position and/or pose information and the current position and/or pose information of the remote end of the mechanical arm at the first coordinate system.

In the step of acquiring the first target position and/or pose information of the remote end of the mechanical arm at the first coordinate system, the control method further includes acquiring motion information of a motion input device; analyzing the motion information to obtain increment position and/or pose information of the remote end of the mechanical arm at the first coordinate system; acquiring the current position and/or pose information of the remote end of the mechanical arm at the first coordinate system; calculating the first target position and/or pose information according to the increment position and/or pose information and the current position and/or pose information of the remote end of the mechanical arm at the first coordinate system.

Before the step of acquiring the first target position and/or pose information of the remote end of the mechanical arm at the first coordinate system and the current position and/or pose information of each of the controlled end instrument at the coordinate system, the control method further includes acquiring description information of a structure feature of the mechanical arm and each of the one or more operating arms having the controlled end instrument which is mounted on at the remote end of the mechanical arm, and generating a model image corresponding to a structure feature of the mechanical arm and corresponding operating arm according to the description information.

After the step of determining if each second target position and/or pose information is effective and acquiring the corresponding determine result, the control method includes changing a color of a model image of the operating arm corresponding to the controlled end instrument if the second target position and/or pose information is determined to be ineffective.

In another aspect, a computer readable storage medium is provided. The computer readable storage medium is configured for storing a computer procedure, and the computer procedure is loaded by one or more processors and configured for carrying out the above steps.

In another aspect, a control device of an end instrument of a surgical robot is provided. The control device includes a storage medium configured for storing a computer procedure, a processor configured for loading and carrying out the computer procedure, and the computer procedure is loaded by the above processor and carries out the above steps.

In another aspect, a surgical robot is provided. The surgical robot includes a control device, a mechanical arm, the mechanical arm has one or more operating arms mounted on a remote end thereof, and the one or more operating arms each has an end instrument. The end instrument includes an image end instrument and/or operating end instrument. The control device is configured for carrying out the above steps.

The present application has the following beneficial effects:

Based on the first target position and/or pose information of the remote end of the mechanical arm at the first coordinate system and the current position and/or pose information of each of the controlled end instruments at the first coordinate system, second target position and/or pose information of each of the controlled end instruments at a second coordinate system can be calculated, thereby controlling the linked movement of the mechanical arm together with the controlled end instruments according to the first target position and/or pose information of the remote end of the mechanical arm and the second target position and/or pose information of the controlled end instrument, such that a current position and/or pose of the controlled end instrument can be maintained unchanged during adjusting the remote end of the mechanical arm.

BRIEF DESCRIPTION OF THE DRAWINGS

FIG. 1 is a schematic structural view of a surgical robot according to an embodiment of the present disclosure.

FIG. 2 is a schematic partial view of the surgical robot of FIG. 1.

FIG. 3 is another schematic partial view of the surgical robot of FIG. 1.

FIG. 4 is a flow chart of a control method of a surgical robot according to an embodiment of the present disclosure.

FIG. 5 is a simple schematic view of the surgical robot in a working state according to an embodiment of the present disclosure.

FIG. 6 is a flow chart of a control method of a surgical robot according to an embodiment of the present disclosure.

FIG. 7 is a flow chart of a control method of a surgical robot according to an embodiment of the present disclosure.

FIG. 8 is a flow chart of a control method of a surgical robot according to an embodiment of the present disclosure.

FIG. 9 shows is a schematic partial view of a working principle of the surgical robot.

FIG. 10 is a schematic view of a resolution of spatial motion angles in a control method of a surgical robot according to an embodiment of the present application.

FIG. 11 is a flow chart of a control method of a surgical robot according to an embodiment of the present disclosure.

FIG. 12 is another flow chart of a control method of a surgical robot according to an embodiment of the present disclosure.

FIG. 13 is a flow chart of the control method of the surgical robot in a one-to-one operating mode according to an embodiment of the present disclosure.

FIG. 14 is a schematic view of the control method of the surgical robot in the one-to-one operating mode according to an embodiment of the present disclosure.

FIG. 15 is a flow chart of the control method of the surgical robot in a two-to-one operating mode according to an embodiment of the present disclosure.

FIG. 16 is a schematic view of the control method of the surgical robot in the two-to-one operating mode according to an embodiment of the present disclosure.

FIG. 17 is a flow chart of a control method of a surgical robot according to an embodiment of the present disclosure.

FIG. 18 is a flow chart of a control method of a surgical robot according to an embodiment of the present disclosure.

FIG. 19 is a schematic view of the control method of the surgical robot in the two-to-one operating mode according to another embodiment of the present disclosure.

FIG. 20 is a schematic structural view of a surgical robot according to another embodiment of the present disclosure.

DETAILED DESCRIPTION

For ease of understanding of the present application, the present application will be described more fully hereinafter with reference to the associated drawings. Preferred embodiments of the present application are set forth in the accompanying drawings. This application may, however, be embodied in many different forms and is not limited to the embodiments described herein. Rather, these embodiments are provided for the purpose of providing a more thorough and thorough understanding of the disclosure of the present application.

It should be noted that when an element is referred to as being "disposed on" another element, it may be directly on the other element or intervening elements may also be present. When an element is considered to be "connected" to another element, it may be directly connected to another element or intervening elements may be present at the same time. When an element is considered to be "coupled" to another element, it may be directly coupled to another element or intervening elements may be present at the same time. As used herein, the terms "vertical", "horizontal", "left", "right" and the like are intended for purpose of illustration only and are not intended to be limiting. As used herein, the terms "remote end" and "proximal end" are common terms in the art of interventional medical devices, where "remote end" refers to the end far away from the user during the surgical procedure, and the "proximal end" refers to the end close to the user during the surgical procedure.

Unless defined otherwise, all technical and scientific terms used herein have the same meaning as commonly understood by one of ordinary skill in the art. The terminology used herein is for the purpose of describing particular embodiments only and is not intended to be limiting of the invention. As used herein, the term "and/or" includes all combinations of one or more of the associated listed items.

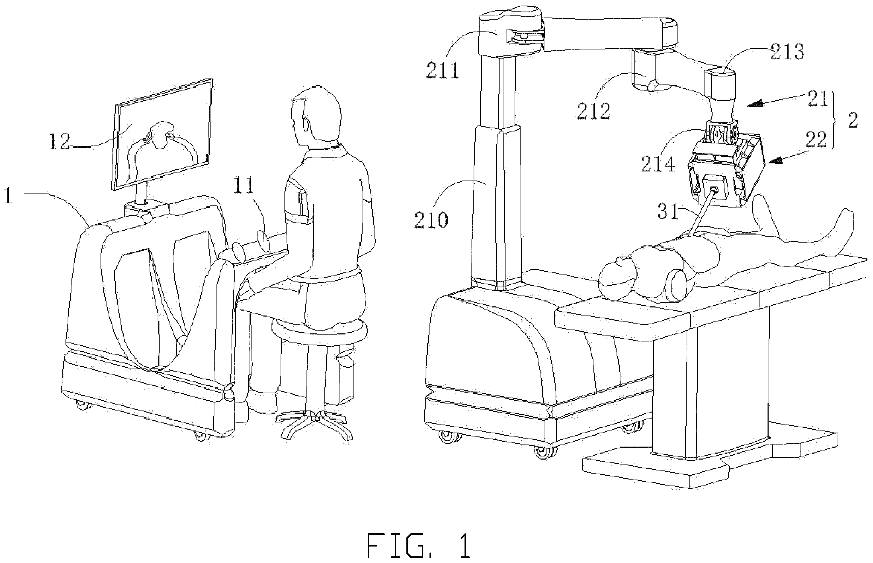

Referring to FIGS. 1 to 3, a schematic structural view of a surgical robot according to an embodiment, and a partial schematic view thereof are illustrated.

A surgical robot includes a master console 1 and a slave operating device 2. The master console 1 has a motion input device 11 and a display 12. A doctor gives a control command to the slave operating device 2 by operating the motion input device 11, to make the slave operating device 2 perform corresponding operation according to the control command of the doctor given to the motion input device 11, and the surgical area is observed by the display 12. In particular, the slave operating device 2 has an arm body mechanism, the arm body mechanism has a mechanical arm 21 and an operating arm 31 detachably mounted on a remote end of the mechanical arm 21. The mechanical arm 21 includes a base and a connection assembly sequentially connected, and the connection assembly has a plurality of joint assemblies. The operating arm 31 includes a link 32, a connection assembly 33, and an end instrument 34, in which the connection assembly 33 may have a plurality of joint assemblies, the operating arm 31 adjusts the pose of the end instrument 34 by adjusting the joint assemblies, and the link 32, the connection assembly 33, and the end instrument 34 are sequentially connected. The end instruments 34 may include an image end instrument 34A and one or more operating end instruments 34B. The mechanical arm 21 and/or the operating arm 31 can move with the motion input device 11.

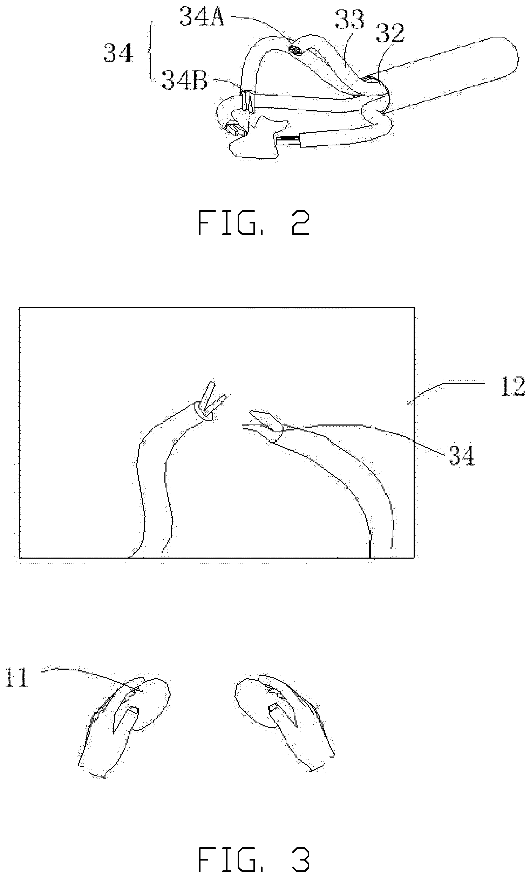

For example, the motion input device 11 can be connected to the master console 1 by a connection wire, or by a rotatable link connected to the master console 1. The motion input device 11 can be configured as a handheld type or wear type (often being wore at a front portion of your wrist, such as a finger or palm), which has a plurality of effective degrees of freedom. Exemplary, the motion input device 11 is configured to be in a form of a joystick shown in FIG. 3. In one case, the number of effective degrees of freedom of the motion input device 11 is configured to be less than the number of the task degrees of freedoms of a remote end of the arm body mechanism; in another case, the number of effective degrees of freedom of the motion input device 11 is configured not to be less than the task degrees of freedoms of the remote end of the arm body mechanism. In order to freely follow movement and rotation of the doctor's hand without constraints, the motion input device 11 is exemplarily configured to have six effective degrees of freedom, in particular, the effective degrees of freedom of the motion input device 11 refer to the effective movement degrees of freedom following the doctor's hand, to make the doctor has a greater free space to operate, and can produce more meaningful data by analyzing each of the effective degrees, satisfying the control of the mechanical arm 21 for almost all configurations.

The motion input device 11 follows movement of the doctor's hand, and collects motion information of the motion input device 11 itself from the hand movement in real time. Using the motion information, the position information, pose information, speed information, and acceleration information can be analyzed. The motion input devices 11 include, but are not limited to, magnetic navigation positioning sensors, optical positioning sensors, or link type master consoles.

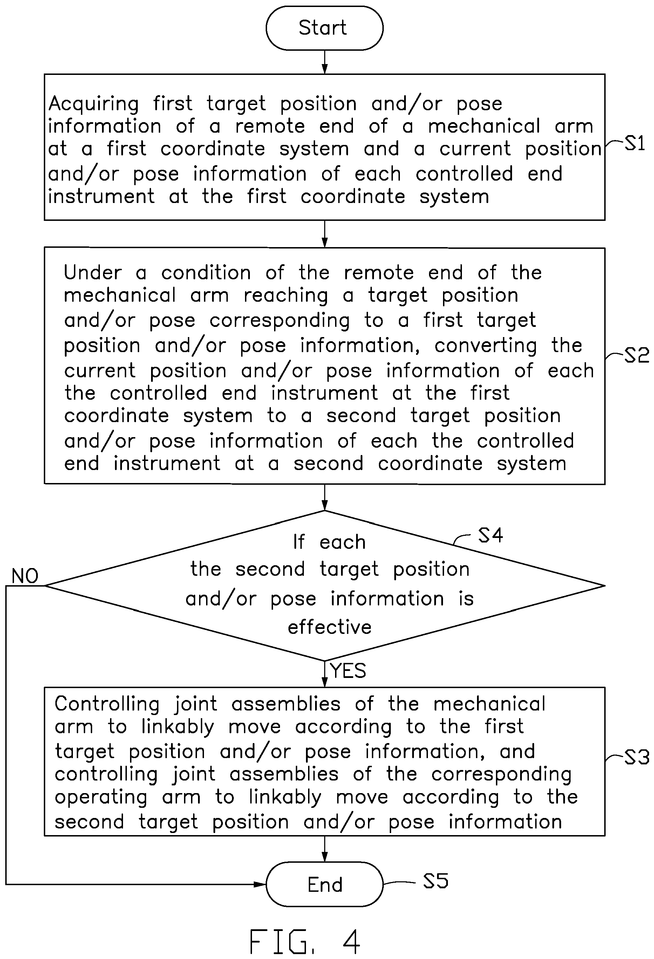

In an embodiment, a control method of an end instrument of a surgical robot is provided. As shown in FIG. 4, the control method includes the following steps:

Step S1, acquiring first target position and/or pose information of a remote end of a mechanical arm in a first coordinate system and current position and/or pose information of each of controlled end instruments in the first coordinate system.

The first coordinate system refers to a base coordinate system of the mechanical arm. The controlled end instruments refer to end instruments need to be passively controlled to maintain a current position and/or pose unchanged. The end instruments may also include one or more non-controlled end instruments, the non-controlled end instrument refers to an end instrument that follows a movement of the mechanical arm but without need to maintain a current position and/or pose unchanged, thus without the need to be passively controlled. Before step S1, a selection instruction input by a user is acquired, and according to the selection instruction, one or more end instruments are selected from the end instruments to be the controlled end instruments. Of course, the system can also default to one or more (including all) end instruments to be the controlled end instruments.

Step S2, under a condition of the remote end of the mechanical arm reaching a target position and/or pose corresponding to the first target position and/or pose information, converting the current position and/or pose information of each of the controlled end instruments at the first coordinate system to obtain second target position and/or pose information of a corresponding controlled end instrument at a second coordinate system.

The second coordinate system refers to a tool coordinate system of the mechanical arm.

Step S3, controlling joint assemblies of the mechanical arm to linkedly move according to the first target position and/or pose information to allow the remote end of the mechanical arm to reach the target position and/or pose corresponding to the first target position and/or pose information, and controlling joint assemblies of the operating arm to linkedly move according to the second target position and/or pose information to allow the controlled end instruments to maintain a current position and/or pose unchanged.

By the above steps S1~S3, the first target position and/or pose information of the remote end of the mechanical arm 21 at the first coordinate system and the current position and/or pose information of each of the controlled end instruments at the first coordinate system are converted to be the second target position and/or pose information of each of the controlled end instruments at the second coordinate system, thereby controlling the linked movement of the mechanical arm 21 and the controlled end instruments 34 according to the first target position and/or pose information and the second target position and/or pose information, thus the controlled end instruments 34 can maintain a current position and/or pose unchanged during adjusting the remote end of the mechanical arm 21, and this operation is convenient and safe.

In one embodiment, in step S2, exemplarily, the second target position and/or pose information of the controlled end instrument 34 at the second coordinate system can be calculated by the following formula:

$$\text{Formula (1): } {}^{T}_{An}T = {}^{B}_{T}T^{-1} \bullet {}^{B}_{An}T$$

In particular, ${}^{T}_{An}T$ refers to the second target position and/or pose information of the controlled end instrument An at the second coordinate system, ${}^{B}_{T}T^{-1}$

refers to the current position and/or pose information of the remote end of the mechanical arm at the coordinate system, B refers to the base coordinate system of the remote end of the mechanical arm, T refers to a tool coordinate system of the mechanical arm. In essence, this formula can be understood by such description, i.e., under the condition of the target position and/or pose corresponding to the first target position and/or pose information of the remote end of the mechanical arm 21 at the first coordinate system, the current position and/or pose information of the controlled end instrument 34 at the first coordinate system can be converted to the second target position and/or pose information of the controlled end instrument 34 at the second coordinate system.

It should be understood that the derivation here is primarily analyzed from the principle, ignoring the matrix conversion relationship of each joint assembly of the mechanical arm 21 and the operating arm 31, and according to this method, those skilled in the art will be easily obtain the second target position and/or pose information of the controlled end instruments 34 at the second coordinate system.

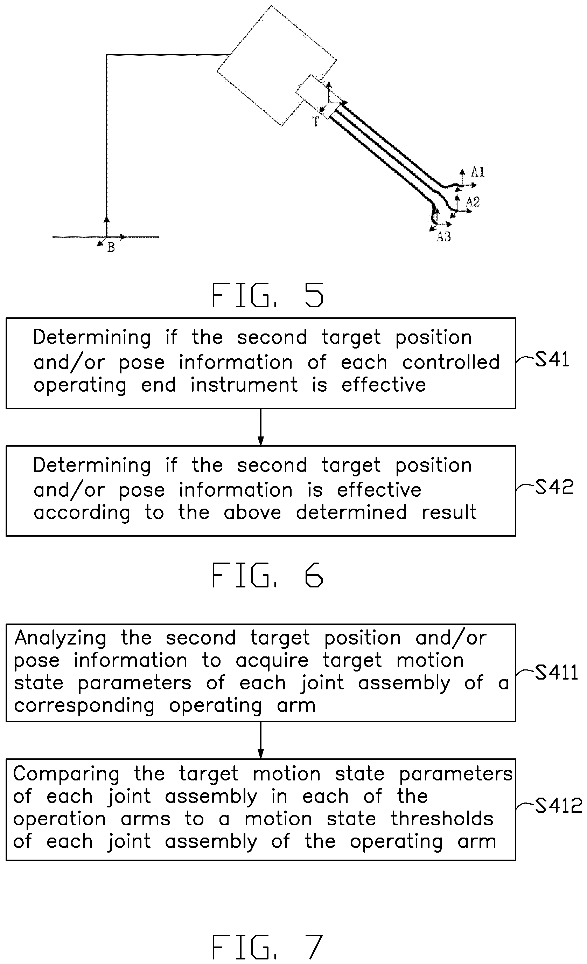

In a simple schematic view of a single port surgical robot in a working state as shown in FIG. 5, a remote end of the mechanical arm 21, for example, may have three operating arms 31 each have an operating end instrument 34B, assuming that the end instruments A1, A2, and A3 of the three operating arms 31 are all configured to be controlled end instruments, and since they are at a common first coordinate system, the respective formulas (1) can be used to generate respective calculation formulas, as follows:

$$\text{Formula (2) } {}^{T}_{A1}T = {}^{B}_{T}T^{-1} \bullet {}^{B}_{A1}T$$

$$\text{Formula (3) } {}^{T}_{A2}T = {}^{B}_{T}T^{-1} \bullet {}^{B}_{A2}T$$

$$\text{Formula (4) } {}^{T}_{A3}T = {}^{B}_{T}T^{-1} \bullet {}^{B}_{A3}T$$

Thereby, according to the formulas (2) to (4), the second target pose information of each of the controlled end instruments A1, A2, and A3 is analyzed, respectively.

In one embodiment, referring again to FIG. 4, after step S2, includes:
Step S4, determining if the second target position and/or pose information of the controlled end instrument is effective (i.e., if it is valid).

In particular, if each second target position and/or pose information is determined to be effective, the control method goes to the step S3; and if one or more of the second target position and/or pose information is determined to be ineffective, the control method goes to step S5.

Step S5, the control goes to end.

That is, the above-described linked movement control is carried out only if the respective second target position and/or pose information is effective. In the case that none of the second target position and/or pose information is effective, the mechanical arm 21 and the operating arm 31 are usually not allowed to be controlled to linkedly move.

In some embodiments, it is possible in the above step S4, the determined effective controlled end instrument 34 can maintain the current position and/or pose unchanged during adjusting the mechanical arm 21, and the determined ineffective controlled end instrument 34 moves with the mechanical arm 21.

In some embodiments, it is also possible if the plurality of second target position and/or pose information is determined ineffective, one of them can be first adjusted to be effective, then according to the adjusted effective second target position and/or pose information and the current position and/or pose information of the controlled end instrument 34 at the first coordinate system to recalculate first target position and/or pose information of the mechanical arm 21 at the first coordinate system. Then according to the adjusted first target position and/or pose information of the mechanical arm 21 at the first coordinate system and current position and/or pose information of the remaining unadjusted controlled end instruments 34 at the first coordinate system to calculate the second target position and/or pose information of them at the second coordinate system using the above formula (1). The above steps can be repeated for cycle calculation until the calculated respective second target position and/or pose information is determined effective; or can set the number of repeat times for the above steps to cycle calculation, if it still does not meet each second target position and/or pose information is effective, the control goes to end.

In one embodiment, as shown in FIG. 6, in particular in the above step S4, includes:

Step S41, determining if the second target position and/or pose information of each of the controlled end instruments is effective.

Step S42, according to the above determined result, determining if the second target position and/or pose information of each of the controlled end instruments is effective.

In particular, as shown in FIG. 7, in the above step S41, includes:
Step S411, analyzing the second target position and/or pose information of the controlled end instrument to obtain a target motion state parameter of each joint assembly of a corresponding operating arm.

In particular, the motion state parameter may be a position parameter, a speed parameter and/or an accelerated speed parameter. The position parameter may be acquired by analyzing the corresponding target position and/or pose information, the speed parameter and/or the accelerated speed parameter may be acquired by calculating the position parameter and the time spent in these position parameters, or instead, the speed parameter and/or the accelerated speed parameter can also be acquired by speed or accelerated speed sensors.

S412, comparing the target motion state parameters of each joint assembly in the operation arm to a motion state threshold of each joint assembly in the operating arm to determine if the corresponding second target position and/or pose information is effective.

During controlling the linked movement of the mechanical arm 21 and the operating arm 31, smoothness and reliability are taken into account, the motion state parameter generally refers to positional parameters, speed parameters, and acceleration parameters, then in step S422, the positional parameters, the speed parameters, and the acceleration parameters of each joint assembly of the corresponding operating arm 31 are respectively compared to a positional parameter threshold, a speed parameter threshold, and an acceleration parameter threshold of the respective joint assembly. Of course, in some embodiments, the motion state parameter can also refer to one or two of the positional parameters, the speed parameters, and the acceleration parameters, for example, the motion state parameter includes only the positional parameters.

By comparing the motion state parameters of each joint assembly of the operating arm 31 with the motion state threshold of each joint assembly based on the adjusted structure characteristics and performance parameters and/or the custom setting, the second target position and/or pose information of the operating arm 31 is determined to be effective or ineffective, thus effectively protecting the operating arm 31 to improve its service life. Exemplary, even by comparing the positional parameter (i.e., motion stroke) of each joint assembly within the positional parameter threshold (i.e., the motion stroke range) of the adjusted object, however, its speed parameter and/or acceleration parameter exceeds the speed parameter threshold/or acceleration parameter threshold, which reflects that its adjustment speed is too fast and/or the speed change is too fast, and it is determined that it may affect safe use, thus is determined to be ineffective.

In particular, if the target motion state parameters of each joint assembly in the operating arm 31 are not greater than the motion state threshold of each joint assembly in the operating arm 31, it is determined that the second target position and/or pose information is effective; and if one of the target motion state parameters of each joint assembly of the operating arm 31 is greater than the motion state threshold of each joint assembly of the operating arm 31, it is determined that the second target position and/or pose information is ineffective.

It is worth noting that, in some situations, when the mechanical arm 21 is in a moving state, it is necessary to ensure that the remote end of the mechanical arm 21 moves around a fixed point (a remote center of motion, RCM), that is, the mechanical arm 21 is in a RCM constraint movement, this can be realized by setting the task degrees of freedom of the remote end of the mechanical arm 21, with the task degrees of freedom may only be related to the degrees of freedom of pose.

The fixed point has a relatively fixed positional relationship with the remote end of the mechanical arm. Depending on the special control purpose, the origin of the second coordinate system in some embodiments may be the fixed point, the origin of the second coordinate system in other embodiments may be a point on the remote end of the mechanical arm.

The detecting unit connected to the processing system can be provided at a connection between the remote end of the mechanical arm 21 and the operating arm 31, which is used to generate a trigger signal for triggering movement of the remote end of the mechanical arm 21 around the fixed point when the trocar is reliably connected to the remote end of the mechanical arm 21. When the trigger signal is detected, the processing system automatically sets the corresponding task degrees of freedom to control the remote end of the mechanical arm 21 to perform the RCM constraint movement. Of course, it is also possible by actively set the corresponding task degrees of freedom by a user to control the remote end of the mechanical arm 21 to perform the RCM constraint movement. In addition, the trigger signal can also be input by the user via the input device such as a button.

Furthermore, depending on the setting, the remote end of the operating arm 31 may have a fixed point, and the fixed point of the operating arm 31 is different from that of the mechanical arm 21, and the end instrument 34 of the operating arm 31 can also be configured to move around the fixed point of the operating arm 31, that is, the RCM constraint movement is performed.

Depending on the configuration of task degrees of freedom of the remote end of the mechanical arm and/or the remote end of the operating arm, RCM constraint control can be performed on the remote end of the mechanical arm and/or the remote end of the operating arm to be suitable to be applied to a variety of use scenarios.

In an embodiment, a processing system is configured to selectively control the controlled end instrument 34 to maintain a position and/or pose unchanged. The specific controls of the controlled end instrument 34 by the processing system can be selected according to the control instruction input by the doctor. The control instruction essentially reflects the doctor's configuration of the task degrees of freedom of the controlled end instrument 34.

The above control of the mechanical arm 21 and the respective operating arms 31 can also be carried out in a lack of view field, i.e., in the absence of an operation arm 31 having an image end instrument 34A. More preferably, the above control can be carried out in the case where there is an operation arm 31 having an image end instrument 34A provided at the remote end of the mechanical arm 21, and this is more suitable for actual operation procedures. Usually, this method does not require control as described above for the operating arm 31 having the image end instrument 34A, but allows the operating arm 31 having the image end instrument 34A directly move follow with the movement of the mechanical arm 21. In this way, the doctor can intuitively observe changes in the field of view due to the adjustment of the mechanical arm 21, that is, the doctor can adjust the field by controlling the remote end of the mechanical arm 21, and utilizing the field of view to have a safe and accurate control of the operating arm 31 having the controlled operating end instrument 34B. In some embodiments, since the controlled operating end instrument 34B can maintain position and/or pose unchanged during adjustment of the image end instrument 34A, the image end instrument 34A and the respective controlled operating end instrument 34B are adjusted by intermittentively, thus facilitating to give the surgical operation more space.

In an embodiment, it is assumed that only the operating end instrument 34B can be controlled as described above, and the image end instrument 34A is allowed to move with the remote end of the mechanical arm 21 to provide a real-time varying field, that is, in the situation of only the operating end instrument 34B can be configured as the above controlled end instrument, before the above step S1, including:

Identifying a type of the end instruments provided on each of the operating arms of the mechanical arm, and after identifying the corresponding operating arm having the controlled operating end instrument, proceeds to step S1.

Specifically, it is necessary to obtain the description information of the respective operating arm 31 when the operating arm 31 is attached to the remote end of the mechanical arm 21, which includes the type information of the end instrument 34 set thereon; after that, according to the acquired description information, identifying the operation arm 31 having the controlled end instrument 34B, which is hereby go to step S1 to automatically perform the desired control of the operating arm 31 having the controlled operating end instrument 34B according to a purpose of the present application. In particular, the description information generally includes a link parameter of the operating arm 31. The acquisition method of the processing system for the description information can be implemented by a data interface or an induction device, and the description information is stored in the chip (including memory, electronic tag, etc.) of the operating arm 31.

In one embodiment, in the above-described step S1, for how to obtain the current position and/or pose information of each of the controlled end instruments 34 at the first coordinate system, example that through acquiring the joint angle of each joint assembly of the respective operation arms 31 and analyzing it by forward kinematics.

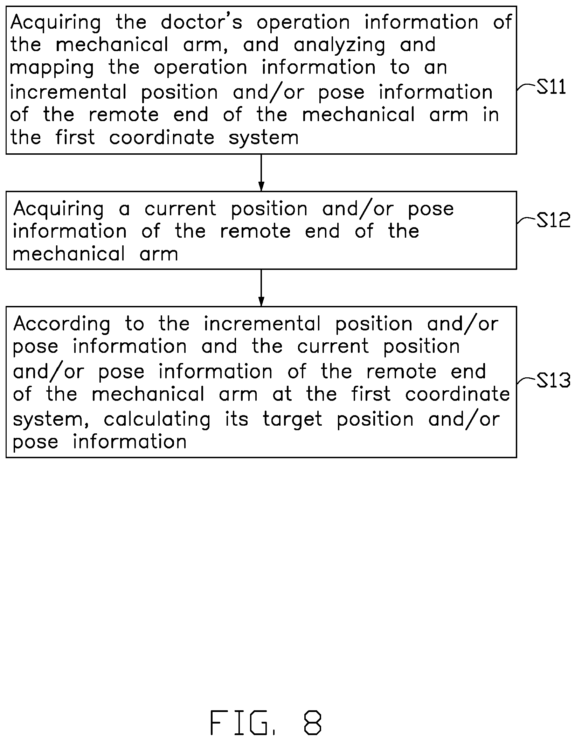

In one embodiment, in the above-described step S1, for how to acquire the first target position and/or pose information of the remote end of the mechanical arm 21 can be performed by the steps shown in FIG. 8.

Step S11, acquiring doctor's operation information of the mechanical arm, and analyzing and mapping the operation information to incremental position and/or pose information of the remote end of the mechanical arm in the first coordinate system.

In particular, the operation information may be external force information (for example, a six-dimensional force/torque vector) generated by the doctor applying an external force to the remote end of the mechanical arm to drag the remote end of the mechanical arm to move; or the operation information can be movement information of the motion input device 11 itself to remotely operate the remote end of the mechanical arm.

Step S12, acquiring current position and/or pose information of the remote end of the mechanical arm at the first coordinate system.

Step S13, according to the incremental position and/or pose information and the current position and/or pose information of the remote end of the mechanical arm at the first coordinate system, calculating its target position and/or pose information.

It should be understood that the method described in the above steps S11 to S13 are also suitable for remote operation of each of the operating arms 31, such as the mechanical arm 21 is not moved, and only control the operating arm 31 to move; or the mechanical arm 21 and the user arm 31 are considered to be a series of structure of an arm mechanism to perform the entire control. These two examples implement the objectives of the invention different from the present application.

The present application mainly takes the operation information as motion information for an example for a detailed description.

Further, the above step S11 is specifically: acquiring motion information input by a motion input device, and analyzing and mapping the motion information to be the incremental position and/or pose information of the remote end at the first coordinate system.

This step is mainly to analyze the motion information at a previous time and at a next time, which may be adjacent times, or may be spaced a time therebetween. In an embodiment, by calculating the position and/or pose change of the motion information at a fixed coordinate system of a next time relative to that of a previous time, thus defining the incremental position and/or pose information. The incremental position and/or information of the fixed coordinate system is then mapped to the incremental position and/or pose information of the end instrument 34 at a reference coordinate system. Exemplary, the fixed coordinate system can be defined at the display, of course, the fixed coordinate system can also be defined in other parts of the surgical robot, which is not movable at least during operation. Among them, "mapping" represents a relationship of corresponding conversion.

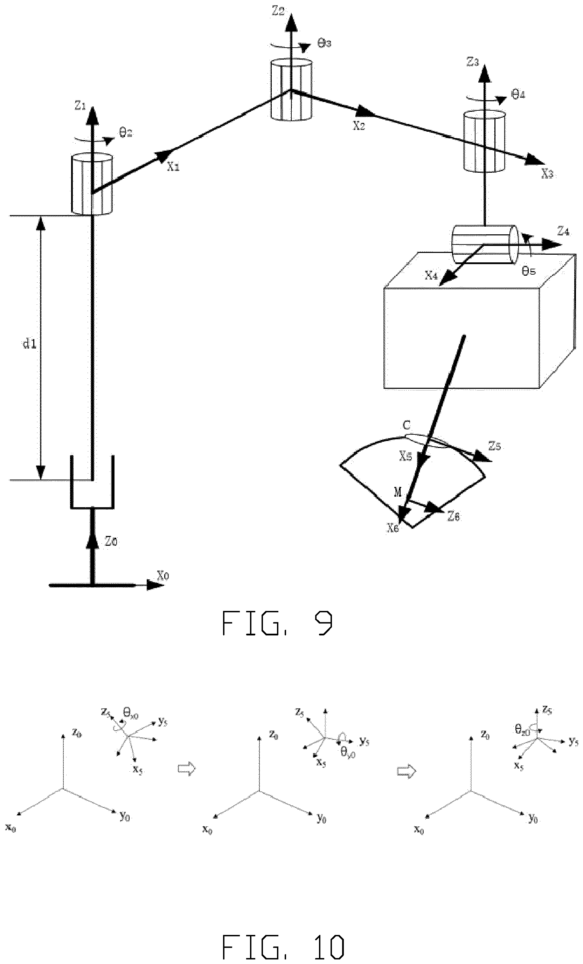

Further, the above step S12 may include: First, obtaining position information of each joint assembly of the mechanical arm. Specifically, the corresponding position information can be acquired by a position sensor such as an encoder installed at the joint assembly. In the embodiment illustrated in FIGS. 1 and 9, the mechanical arm 21 has 5 degrees of freedom, and can be collected such a group of position information by means of each of the position sensors (d1, θ2, θ3, θ4, θ5).

Then, calculating the current position and/or pose information of the mechanical arm according to the position information of each joint assembly. In particular, it is usually possible to calculate by combining forward kinematics. A kinematics model of a fixed point of the mechanical arm 21 (i.e., at the C point, the origin of the tool coordinate system of the robot arm 21 is at the fixed point) is established to the base of the mechanical arm 21, then the model conversion matrix ${}^0_C T$ of the C point and the base is output. The calculation method may be

$$ {}^0_C T = {}^0_1 T\, {}^1_2 T\, {}^2_3 T\, {}^3_4 T\, {}^4_C T $$

.

According to the model conversion matrix ${}^0_C T$ of the C point to the base, the position and/or pose information of the C point at the fixed coordinate system is acquired. It is assumed that without changing the C point position, rotating the coordinate system of the C point, to reach its pose described by the model conversion matrix, and to obtain a rotary axis angle [θx0 , θy0 , θz0 ], as shown in FIG. 10. Wherein θx0 refers to a rolling angle, θy0 refers to a yaw angle, θz0 refers to a pitch angle, in the mechanical arm 21 shown in FIG. 9, the degrees of freedom of the rolling angle are lacked, thus θx0 is actually not adjustable.

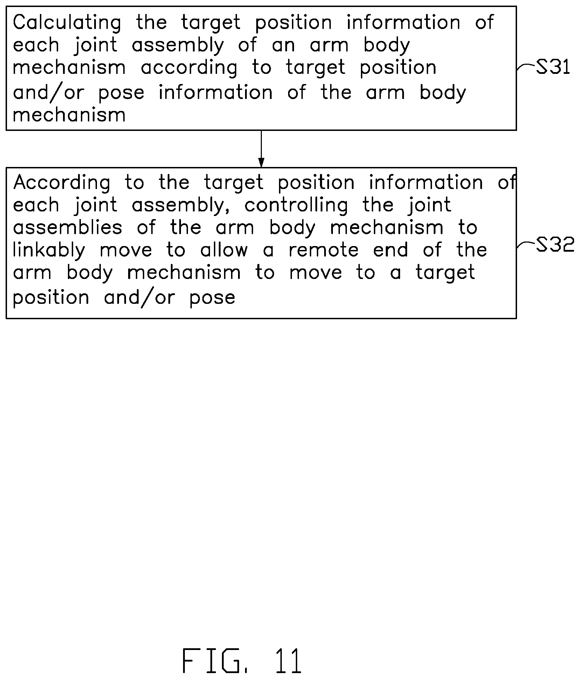

In one embodiment, as shown in FIG. 11, the linked movement of the remote end of the mechanical arm 21 and the controlled end instrument 34 can be realized by a position control method. Specifically, in step S3, including:

Step S31, calculating target position information of each joint assembly of an arm body mechanism according to target position and/or pose information of the arm body mechanism.

In particular, it is usually possible to calculate inverse kinematics.

Step S32, according to the target position information of each joint assembly, controlling joint assemblies of the arm body mechanism to linkedly move to allow a remote end of the arm body mechanism to move to a target position and/or pose.

It should be understood that the arm body mechanism herein can be a mechanical arm 21 and/or an operating arm 31, and each of the mechanical arm 21 and the operating arm 31 is performed according to steps S31- S32.

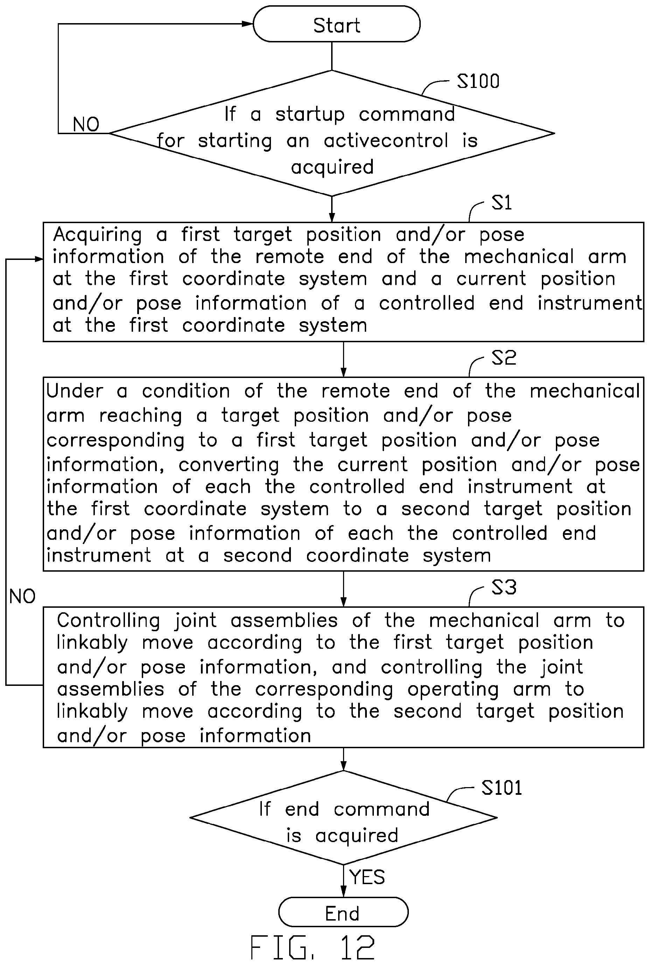

Further, as shown in FIG. 12, in order to reflect whether the active control of the arm body mechanism is the doctor's actual intention, before step S1, the control method can carry out:

Step S100, detecting whether a startup command for starting an active control is acquired.

In particular, the start command can be input by an active control switch. After the acquiring of the startup command is detected, go to the above-described step S1.

Further, after step S3, the control method performs:

Step S101, detecting whether an end command is acquired to stop the active control.

In particular, the end command can also be entered by the active control switch. After the acquiring of the end command is detected, the control of the arm body is ended; otherwise, reenter into steps S1 to S101.

The above active control switch can be configured as a foot switch, a button switch, a master console switch, and the like.

Furthermore, in order to be more close to the actual intention of the doctor's active control for the arm body, before step S1, it can be performed:

Detecting whether the surgical robot is in a doctor's operating state.

This step can be implemented by setting a proximity switch in the master console to detect whether a head is close to is detected. When the head close to is detected by the proximity switch, the start condition is satisfied, thereby proceeding to the step S1. Alternatively, it is also possible to set a speech recognition module, then according to doctor's startup speech to confirm the startup of the command, thereby proceeding to the step S1.

In an embodiment, the configuration of the task degrees of freedom can be performed for the remote end of the mechanical arm 21 and each of the controlled end instruments 34 to generate the configuration information, and the configuration information of the task degrees of freedom of each of controlled end instruments 34 is usually configured to be the same.

The configuration of task degrees of freedom for the remote end of the mechanical arm 21 can be understood to be enable functionality, i.e., the movement degrees of freedom of the mechanical arm 21 is allowed. For example, in the aforementioned step S11, combining the configuration information of the task degrees of freedom in the remote end of the mechanical arm 21, an operation information (i.e., an external force information or a motion information) is analyzed and mapped to the incremental position and/or pose information of the mechanical arm 21 at the first coordinate system. In particular, the task degrees of freedom can be set by the user according to a field of use, i.e, the user sets the position and/or pose of the remote end of the mechanical arm according to the requirements.

The configuration of the task degrees of freedom of controlled end instrument 34 can be understood as a disabled function, i.e., disable the degrees of freedom of the end instrument 34, combined with the above description, the position and/or pose of the controlled end instrument 34 can be maintained unchanged by this configuration. For example, in the foregoing step S12, each of the second target position and/or pose information is calculated in conjunction with the configuration information of the task degrees of freedom of the controlled end instrument 34.

It should be understood that, in other embodiments, for example, in the case where the intermittent action described in the foregoing is performed to control the mechanical arm 21 and the operating arm 31 to move, especially when each of the operating arms 31 is remotely operated respectively, each controlled end instrument 34 is reconfigured the same or different task degrees of freedom to achieve different inventive purposes, at this time, in order to ensure safety, it usually requires a doctor to remotely operate these operating arms 31 in a condition of the doctor has a view field.

The above configuration of the task degrees of freedom of the remote end of the mechanical arm 21 and the end instrument 34 can be freely configured by a doctor, such as by a human-machine interaction interface having predefined task degrees of freedom.

Specifically, the task degrees of freedom of the remote end of the arm body mechanism (mechanical arm 21 and/or user arm 31) can be understood to be allowed movement task degrees of freedom of the remote end of the arm body mechanism in a Cartesian space, which is at most to be 6 degrees of freedom. The degrees of freedom of the remote end of the arm body mechanism in the Cartesian space are effective degrees of freedom, and the effective degrees of freedom of the remote end of the arm body mechanism is related to its configuration (i.e., structural features), and the effective degrees of freedom of the remote end of the arm body mechanism can be understood to be the degrees of freedom of the remote end of the arm body mechanism in the Cartesian space. The task degrees of freedom of the end instrument 34 can be configured are associated with the effective degrees of freedom of the mechanical arm 21 and the operating arm 31, in which the mechanical arm 21 and the operating arm 31 can be considered a series arm body mechanism, both together to provide the task degrees of freedom configured for the end instrument 34, and the same it is at most 6 degrees of freedom. The configuration information of the task degrees of freedom of the remote end of the arm body mechanism is which movement freedom permitted to the remote end of the arm body.

A utilization of the configuration information of the task degrees of freedom of the remote end of the arm body mechanism, can be understood to be utilized in step S11, or be utilized when mapping. For example, it can be configured to allow movements in [x, y, z] these three degrees of freedom in the position and/or pose information, in any form of utilization, mainly presented in the final target position and/or pose information, i.e., the [x, y, z] three degrees of freedom follow change of an operation information such as motion information or external force information, but the [$\alpha$, $\beta$, $\gamma$] three degrees of freedom do not follow change of the motion information or external force information. In particular, x refers to horizontal moving task degrees of freedom, y refers to vertical movement task degrees of freedom, z refers to front and rear moving task degrees of freedom, $\alpha$ refers to a yaw angle task degrees of freedom, $\beta$ refers to a pitch angle task degrees of freedom, and $\gamma$ refers to a rolling angle task degrees of freedom.

In the mechanical arm 21 as shown in FIG. 9, the effective degrees of freedom of the mechanical arm 21 includes [x, y, z, $\alpha$, $\beta$], according to the structural characteristics of the joint assemblies 210 to 214 in the mechanical arm 21, in the rolling angle $\gamma$ the mechanical arm 21 does not have any degree of freedom. The task degrees of freedoms of the power mechanism 22 which is connected to the remote end of the mechanical arm 21 and configured for mounting and driving the operating arm 31 having the end instrument 34, is selected from the effective degrees of freedom of the mechanical arm 21.

If the configuration information of the task degrees of freedom of the power mechanism 22 (i.e, the degree of freedom of the remote end of the mechanical arm 21) is [x, y, z, $\alpha$, $\beta$], and the configured information of the task degrees of freedom of the power mechanism 22 is completely matched with the effective degrees of freedom information of the mechanical arm 21, at which state the power mechanism 22 is freely controlled, and the power mechanism 22 is controlled to be movable in a large range to adapt to the operating room arrangement.

When the configuration information of the task degrees of freedom of the power mechanism 22 is [x, y, z, $\alpha$] or [x, y, z], etc., the configuration information of the task degrees of freedom of the power mechanism 22 is included in, but not completely matched with the effective degrees of freedom information of the mechanical arm 21. In controlling the power mechanism 22, only the corresponding degrees of freedom in [x, y, z, $\alpha$] or [x, y, z] can be adjusted. When the power mechanism 22 is in constraint control, the power mechanism 22 can be controlled within a predetermined range.

In particular, if the configuration information of the task degrees of freedom of the power mechanism 22 only includes [$\alpha$, $\beta$], this belongs to the RCM constraint control in the constraint control, that is, moving around the remote motion center (i.e., the fixed point), only the yaw angle and the pitch angle can be adjusted, and they can meet a fine adjustment requirement during the operation.

Of course, when the effective degrees of freedom information of the mechanical arm 21 includes [x, y, z, $\alpha$, $\beta$, $\gamma$], and by the configuration of the task degrees of freedom of the power mechanism 22, the RCM constraint control can include such control types as only for the yaw angle, only for the pitch angle, only for the rolling angle, for both of the yaw angle and the pitch angle, for both of the yaw angle and the rolling angle, and for both of the pitch angle and the rolling angle, and for all of the yaw angle, the pitch angle, and the rolling angle.

In one configuration, if the configuration information of the task degrees of freedom of the power mechanism 22 is partially included in the effective degrees of freedom information of the mechanical arm 21, a preferred choice is to prompt the configuration error, another choice is only part of the degrees of freedom contained in the effective degrees of freedom of the mechanical arm 21 is adjustable. Take the mechanical arm 21 shown in FIG. 9 as an example, if the configuration information of the task degrees of freedom of the power mechanism 22 is [y, z, $\alpha$, $\beta$, $\gamma$] or [x, y, z, $\alpha$, $\beta$, $\gamma$], on one hand, it can be prompted the configuration error information, and the other hand, allowing corresponding degrees of freedom in [y, z, $\alpha$, $\beta$] or [x, y, z, $\alpha$, $\beta$] to be adjustable. This can be configured according to actual requirement.

Surgical robots can provide one or more motion input devices 11. In an embodiment, the surgical robot provides two motion input devices 11. To facilitate operation, the two motion input devices 11 are provided to both hands, which can be manipulated one people, or two people. The mechanical arm 21 can selectively follow one motion input device or two motion input devices, i.e., the mechanical arm 21 can follow any of the two motion input devices 11, defining a one-to-one operating mode with a motion input device 11 to control a mechanical arm 21, or defining a two-to-one operating mode with two motion input devices 11 to control a mechanical arm 21. When controlling a mechanical arm 21, the one-to-one operating mode or the two-to-one operating mode can be selected. For the one-to-one operating mode, it is possible to further select which motion input device to be controlled.

In an embodiment, for the one-to-one operating mode, by formula $P_n = KP_n$, position and/or pose information P of a motion input device 11 selected to be operated at an n time is acquired, wherein, K refers to a proportional coefficient, usually, K>0, preferably, $1 \geq K > 0$ to achieve zoom of a pose, and easy to control.

In one embodiment, for the two-to-one operating mode, by formula $P_n = K_1 P_{nL} + K_2 P_{nR}$, position and/or pose information P of two motion input devices 11 selected to be operated at an n time is acquired, wherein usually, $K_1 > 0$, $K_2 > 0$; preferably, $1 \geq K_1 > 0$, $1 \geq K_2 > 0$.

When calculating the incremental position and/or pose information $\Delta p_{n\_n-1}$ of the motion input device 11 corresponding to the two-to-one operating mode or the two-to-one operating mode at a previous time and at a next time, it can be calculated according to the following formula:

$$\Delta p_{n\_n-1} = P_n - P_{n-1}$$

Of course, it is usually possible to achieve a mapping of the incremental position and/or pose information of the remote end of the mechanical arm 21 at the fixed coordinate system to be the incremental position and/or pose information of the remote end of the mechanical arm 21 at the first coordinate system in conjunction with the task degrees of freedom of the mechanical arm 21.

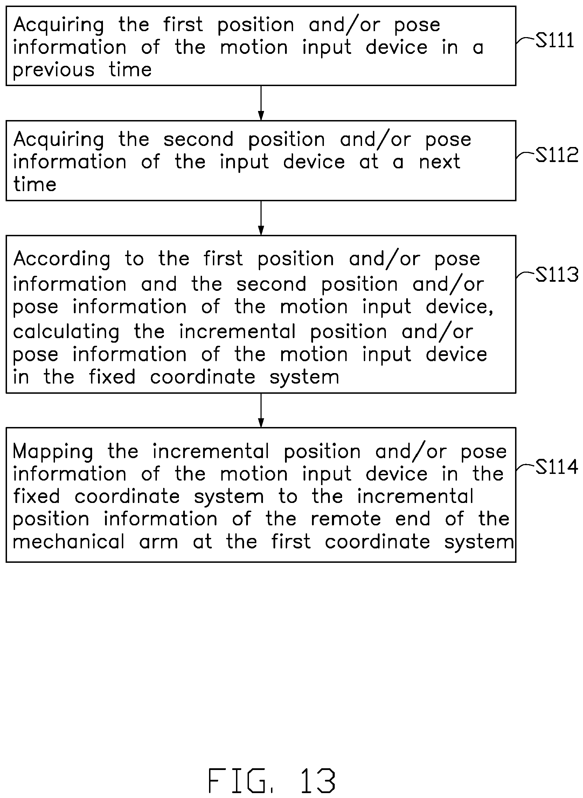

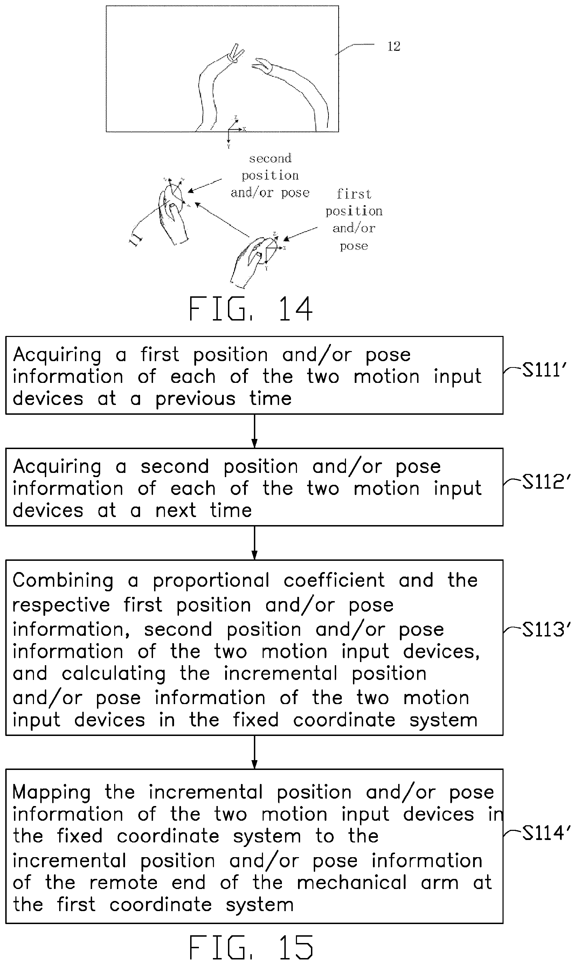

In one embodiment, as shown in FIGS. 13 and 14, for the one-to-one operating modes, the motion information input by the motion input device is acquired, and the motion information is analyzed and mapped to be the incremental position and/or pose information of the remote end of the mechanical arm in the first coordinate system, which step includes:

    Step S111, acquiring first position and/or pose information of the motion input device at a previous time.

    Step S112, acquiring second position and/or pose information of the motion input device at a next time. In particular, the next time can usually be understood as the current moment, as the time goes, the next time goes to be a previous time of a further next time. In step S111 and step S112, both acquired are position and/or pose information of the motion input device selected for one-to-one operating mode.

    Step S113, according to the first position and/or pose information and the second position and/or pose information of the motion input device, calculating incremental position and/or pose information of the motion input device in a fixed coordinate system.

    Step S114, mapping the incremental position and/or pose information of the motion input device in the fixed coordinate system to be the incremental position and/or pose information of the remote end of the mechanical arm at the first coordinate system.

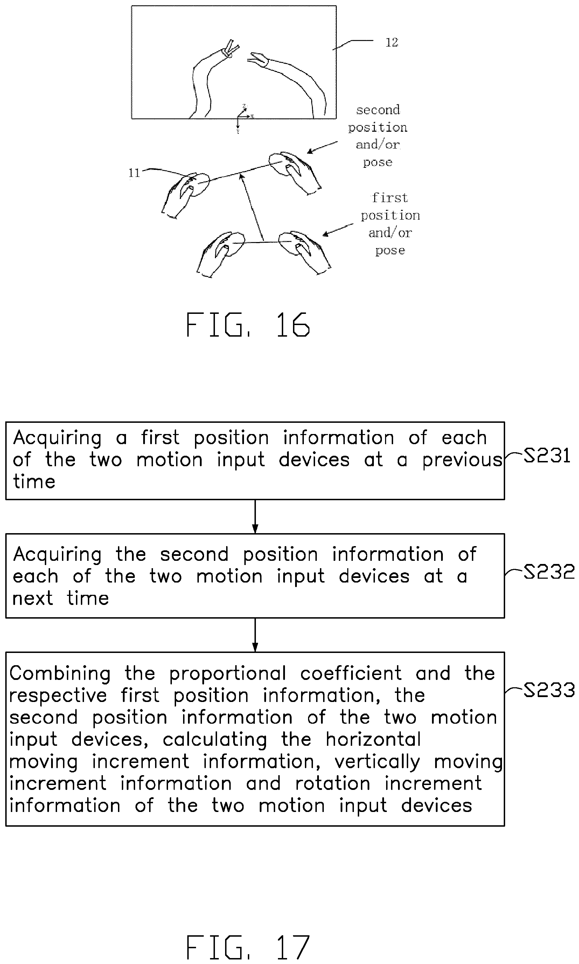

For incremental position and/or pose information in an embodiment, in connection with FIGS. 15 and 16, for the two-to-one operating mode, the motion information input by the motion input device is acquired, and the motion information is analyzed and mapped to be the incremental position and/or pose information of the remote end in the first coordinate system of the mechanical arm, which step includes:

    Step S111', acquiring first position and/or pose information of each of the two motion input devices at a previous time.

    Step S112', acquiring second position and/or pose information of each of the two motion input devices at a next time.

    Step S113', combining a proportional coefficient and the respective first position and/or pose information, second position and/or pose information of the two motion input devices, to calculate the incremental position and/or pose information of the two motion input devices in the fixed coordinate system.

In particular, for step S113', it can be implemented specifically by the following steps:
Calculating incremental position and/or pose information of the first position and/or pose information and the second position and/or pose information of a motion input device at a fixed coordinate system, and calculating another incremental position and/or pose information of the first position and/or pose information and the second position and/or pose information of another motion input device at the fixed coordinate system.

Combining a proportional coefficient to calculate the incremental position and/or pose information of the motion input device in the fixed coordinate system and the incremental position and/or pose information of another motion input device in the fixed coordinate system, to respectively obtain the incremental position and/or pose information of the two motion input devices in the fixed coordinate system.

Step S114', mapping the incremental position and/or pose information of the two motion input devices in the fixed coordinate system to be the incremental position and/or pose information of the remote end of the mechanical arm at the first coordinate system.

In particular, in the two-to-one operating mode, exemplary, the proportional coefficients K1 and K2 both valued 0.5, the acquired incremental position and/or pose information refers to the incremental position and/or pose information of the central points of the two motion input devices. According to the actual situation, K1 and K2 can have other values.

Furthermore, if consideration of the configuration information of the task degrees of freedom in the remote end of the mechanical arm 21 is needed, on one hand, in step S113 (or step S113') can only acquire the position and/or pose information of the degrees of freedom of the motion input device 11 associated with the task degrees of freedom of the remote end of the mechanical arm 21, and then proceeds S114 (or step S114'). On the other hand, it is also possible to acquire a position and/or pose information of all effective degrees of freedom of the motion input device in step S113 (or step S113'), but in step S114 (or step S114'), in the incremental position and/or pose information at the fixed coordinate system, those position and/or pose information of degrees of freedom associated with the task degrees of freedom of the remote end of the mechanical arm 21 is mapped to the incremental position and/or pose information of the remote end of the mechanical arm 21 in the first coordinate system, while remaining the position and/or pose information of the degrees of freedom which is not associated with the task degrees of freedom of the remote end of the mechanical arm 21.

In an embodiment, it is possible to perform step S114 or step S114', as follows:

According to the different types of parameters contained in the acquired incremental position and/or pose information (related to task degrees of freedom), different parameters are corrected by different methods. For example, different types of parameters are corrected by setting different correction factors, the parameters before and after the correction can be expressed as a multiplication relationship; or can set different compensation values to correct different types of parameters, and the parameters before and after correction can be expressed as the relationship between an addition and subtraction; or, the different types of parameters can be corrected in conjunction with the setting correction coefficient and the compensation value, and the parameters before and after the correction may be expressed as the relationship between the multiplication and subtraction.

The steps to correct the different parameters by different methods can be performed in any step between steps S111 to S 114 (steps S111' to S 114'). For example, it can be performed in step S114 (or step S 114'). This step can more accurately reflect the doctor's operation intention to reduce the erroneous operation influence, and compensate for problems that are unreachable due to partial rotational angles due to hand-flexible factors, and adjustable an arbitrary angle.

It is worth noting that because one-to-one operating mode and the two-to-one operating mode are different in habits or adaptability, even if the same correction method is used to correct the different types of parameters in increment position and/or pose information, different correction factors and/or compensation values can be set for the two operating modes.

The one-to-one operating mode and the two-to-one operating mode are both suitable for the case of different task degrees of freedoms of the remote end of the mechanical arm. Consideration the perspective of convenience and accuracy, the one-to-one operating mode is more suitable for the mechanical arm which has more task degrees of freedoms (such as 4 task degrees of freedoms), the two-to-one operating mode is more suitable for the mechanical arm in the case that has less freedom degrees (such as less than 3 task degrees of freedoms).

For example, when using the two-to-one operating mode, it is easy to accurately perform RCM constraint control. At this time, only the position information included in the motion information is analyzed and mapped to be the pose information of the mechanical arm 21, and thus it is easy to utilize the two motion input devices 11 to control the above.

For example, the translational motion information of the two motion input devices 11 in a horizontal direction can be analyzed and mapped to be the yaw angle incremental information of the mechanical arm 21 at the first coordinate system, and the translational motion information of the two motion input devices 11 in the vertical direction is analyzed and mapped to be the pitch angle increment information of the mechanical arm 21 at the first coordinate system. The rotation motion of the two motion input devices 11 on any plane, such as the vertical plan are analyzed and mapped to be the rolling angle increment information of the mechanical arm 21 at the first coordinate system, exemplary, as shown in FIG. 17, the horizontal movement increment information, vertical movement increment information and rotation increment information of two moving input device 11 can be processed by the following steps:

Step S231, acquiring first position information of each of the two motion input devices at a previous time respectively.

Step S232, acquiring second position information of each of the two motion input devices at a next time respectively.

Step S233, combining the proportional coefficient and the respective first position information, the second position information of the two motion input devices, calculating horizontal moving increment information, vertically moving increment information, and rotation increment information of the two motion input devices at the fixed coordinate system.

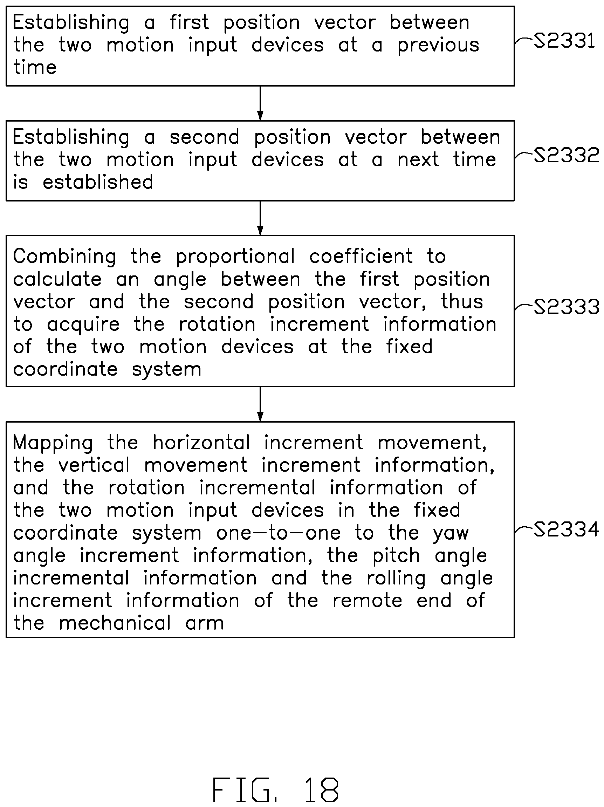

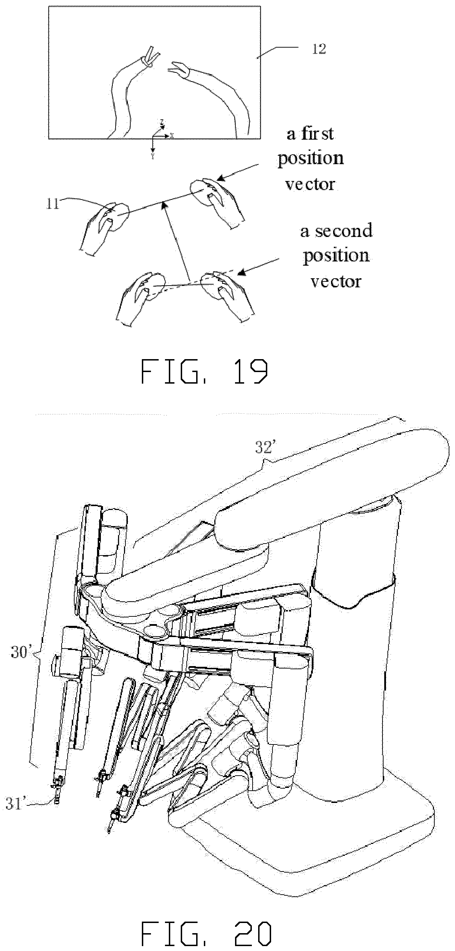

Among them, in step S233, the horizontal moving increment information of the fixed coordinate system and the vertical movement increment information can be calculated with reference to the above-description, and the rotation increment information of the fixed coordinate system can be calculated by the following method, specifically as shown in FIG. 18 and FIG. 19:

Step S2331, establishing a first position vector between the two motion input devices at a previous time.

Step S2332, establishing a second position vector between the two motion input devices at a next time.

Step S2333, combining the proportional coefficient to calculate an angle between the first position vector and the second position vector, thus acquiring the rotation increment information of the two motion input devices at the fixed coordinate system.

Step S2334, mapping the horizontal increment movement, the vertical movement increment information, and the rotation incremental information of the two motion input devices in the fixed coordinate system one-to-one to the yaw angle increment information, the pitch angle incremental information, and the rolling angle increment information of the remote end of the mechanical arm.

In particular, an input device can be configured in the master console and/or the slave operating device, which is used to output a control command for switching mapping relationships. For example, the mapping relationships include natural mapping relationships and nonnatural mapping relationships.

In particular, a natural mapping relationship can be defined as, the incremental position and/or pose information at the fixed coordinate system acquired by analyzing motion information, is one-to-one mapped according to the type of the parameters to be incremental position and/or pose information of the remote end of the mechanical arm 21 at the first coordinate system. Specifically, the horizontal movement increment information of the fixed coordinate system is mapped to be the horizontal movement increment information of the mechanical arm 21 at the first coordinate system, the vertical movement increment information of the fixed coordinate system is mapped to the vertical movement increment information of the mechanical arm 21 in the first coordinate system, the front and rear movement increment information at the fixed coordinate system is mapped to the front and rear movement increment information of the mechanical arm 21 in the first coordinate system, the yaw angle increment information of the fixed coordinate system is mapped to the yaw angle increment information of the mechanical arm 21 at the fixed coordinate system, the pitch angle increment information at the fixed coordinate system is mapped to the pitch angle increment information of the mechanical arm 21 at the first coordinate system, and the rolling angle increment of the fixed coordinate system is mapped to the rolling angle increment information of the mechanical arm 21 at the first coordinate system. These can be selected according to the configuration information of the task degrees of freedom of the mechanical arm 21.

A non-natural mapping relationship is a mapping relationship other than the natural mapping relationship. In an example, the non-natural mapping relationship includes, but is not limited to, a conversion mapping relationship, which includes, but is not limited to, the aforementioned horizontal movement increment information of the fixed coordinate system, vertical movement increment information and rotation increment information one-to-one mapping to be the yaw angular increment information, pitch angle increment information, and rolling angle increment information of the mechanical arm 21. The non-natural mapping relationship can be more easily controlled to the mechanical arm 21 in some cases such as two-to-one mode of operation.

Combined with the configuration information of the acquired task degrees of freedom of the mechanical arm 21, and/or the type information of the operation mode, and/or the type information of the mapping relationship, the motion information is analyzed and mapped to be the position and/or pose incremental information of the remote end of the mechanical arm 21 in the first coordinate system. Furthermore, the doctor can have other modes which is easier to be understood and more convenient to operate according to their habits.

In some embodiments, the number of the effective degrees of effective freedom in the motion input device 11 may be less than 6, however, as long as the effective degrees of freedom of the motion input device 11 is not less than the task degrees of freedoms of the mechanical arm 21, one or more of the above methods may be employed to analyze the motion information and map it to be the position and/or pose incremental information of the mechanical arm 21 in the first coordinate system to achieve the control purpose.

In an embodiment, according to the accuracy of the adjusting process of the arm body mechanism (e.g., a mechanical arm), it can be divided into coarse adjustment mode and fine adjustment mode. The two adjustment modes are both suitable for the aforementioned control methods. In the coarse adjustment mode, the doctor can first control the remote end of the arm body mechanism move to an approximate target position and/or pose, and then switch to the fine adjustment mode, thereby controlling the remote end of the arm mechanism move to an accurate target position and/or pose. More specifically, the difference between coarse adjustment mode and fine adjustment mode is, from the adjustment amplitude or speed of the arm mechanism, the fine adjustment mode presents smaller adjustment amplitude or slower speed compared to that of the coarse adjustment mode, thus facilitating an accurate adjustment for a doctor. Specifically, whether the one-to-one operating mode or the two-to-one operating mode, in the correction in the above step S2011 according to the operating mode and the parameter information type (associated with the degrees of freedom) in the position and/or pose incremental information at the fixed coordinate system, the correction coefficient and/or compensation value of each parameter information in position and/or pose incremental information at the fixed coordinate system is reconfigured, wherein the correction coefficient and/or the compensation value in the fine adjustment mode is usually smaller than the correction coefficient and/or the compensation value in the coarse adjustment mode.

Similarly, to achieve switching between coarse adjustment mode and fine adjustment mode, an input device can be configured on the master console and/or the slave operation device, which is used to output a switching command for switching the adjustment modes.

Preferably, during the doctor adjusting the remote end of the arm body mechanism to the target position and/or pose, if it is found that more than one vector has reached the target position, the task degrees of freedom of the remote end of the arm body mechanism can be reconfigured, for only adjusting the vector that does not reach the target position posture. Exemplary, the surgical robot includes an input device configured to generate a control command for generating task degrees of freedom of reconfiguring the remote end of the arm body. Processing systems are configured to when a control command is acquired, regenerate information of task degrees of freedom of the remote end of the arm body mechanism which is freely configured. In particular, the regenerated information of the task degrees of freedom of the remote end of arm body mechanism freely configured is fully matched with the configuration information of the task degree of freedom of the arm body mechanism configured at the previous time. That is, it is assumed that the information of the task degrees of freedom in the remote end of the arm mechanism is [x, y, z, $\alpha$, $\beta$], however, at a previous time, the arm body mechanism is configured only [x, y, z, $\alpha$], then the information of the new task degrees of freedom of the remote end of the freely configured arm body mechanism generated according to the control command is only [x, y, z, $\alpha$].

For example, if the target position and/or pose needs [x, y, z, $\alpha$, $\beta$, $\gamma$] to be adjusted, if the doctor finds x, y vectors have reached a desired value, the above control command can be triggered and according to the generated new task degrees of freedom information of the arm body mechanism to reconfigure the task degrees of freedom of the remote end of the arm body mechanism to be [z, $\alpha$, $\beta$, $\gamma$], to utilize the aforementioned control method of the arm body mechanism to continue adjusting z, $\alpha$, $\beta$, $\gamma$. Furthermore, if there is a difficulty of adjusting [z, $\alpha$, $\beta$, $\gamma$], reconfigure the task degrees of freedom of the remote end of the arm body mechanism and then utilize the aforementioned control method to one by one adjust the [z, $\alpha$, $\beta$, and $\gamma$] until the arm body mechanism finally completely moves to the target position and/or pose. The input devices configured in the master console (including motion input device 11) and/or the slave operating device include, but are not limited to touch screens, buttons, knobs, pedals, and speech recognition modules. They can be used in combination, or they can be used alone; they can use the same one, or multiple of them. For example, the input device is mostly disposed at the master console, making it easy for the doctor to operate without leaving his current position. For example, the input device can use the speech recognition module to generate and output the corresponding control command to select the corresponding mode according to the speech of the doctor, so that the structure of the surgical robot is relatively simple, and the hands and feet can be free, thus a more continuous (i.e., not interrupting a current operation) operation can be performed.

The method described above is suitable for controlling the image end instrument 34A and the operating end instrument 34B to keep the controlled end instrument maintain the current position and/or the pose unchanged.

The above embodiments are suitable for controlling end instruments in the surgical robot type shown in FIG. 1. The type of surgical robot includes a mechanical arm 21 and more than one operating arms 31 each having an end instruments 34 at the remote end of the mechanical arm 21, with the mechanical arm 21 and the operating arms 31 both have several degrees of freedom.

The above embodiments are also applicable to control of end instruments in the surgical robot type shown in FIG. 20. This type of surgical robot includes a main arm 32', one or more adjustment arms 30' mounted at the remote end of the main arm 32', and one or more operating arms 31' each having an end instrument mounted at the remote end of the adjustment arms 30', with the main arm 32', the adjustment arm 30', and the operating arm 31' each have several degrees of freedom. As shown in FIG. 20, in the surgical robot, there may be four adjustment arms 30', and each of the adjustment arms 30' can have only one operating arm 31'. According to the actual use scenario, the three-section arm structure of the surgical robot as shown in FIG. 20 can be configured to be the two-section arm structure of the surgical robot type shown in FIG. 1 to achieve control. In an embodiment, in the case where the concept of the operating arms in the two types of surgical robots is consistent, for example, according to the configuration, each adjustment arm 30' in the surgical robot of the type shown in FIG. 20 can be regarded as the mechanical arm 21 in the surgical robot type shown in FIG. 1 to perform control; another example, according to the configuration, any of the adjustment arms 30' and the main arm 32' as an entire in the surgical robot type shown in FIG. 20 can be regarded to be the mechanical arm 21 in the surgical robot type shown in FIG. 1 to achieve control. In an embodiment, the main arm 32' in the surgical robot type shown in FIG. 20 can be considered as a mechanical arm 21 in the surgical robot type shown in FIG. 20. The adjustment arm 30' and its corresponding operating arm 31' as an entire in the surgical robot type shown in FIG. 20 can be regarded to be the operating arm 31 in the surgical robot type shown in FIG. 1 to achieve control.

In an embodiment, the control method of the above-mentioned surgical robot is generally configured to be implemented in the control device of the surgical robot, the control device includes a memory and more than one processor, the memory is configured for storing a computer program, a processor is configured for loading and executing the computer program to carry out the control method described in any of the above embodiments.

In an embodiment, a computer readable storage medium is provided, and a computer readable storage medium stores a computer program, which is configured to perform the control method steps described in any of the above-described embodiments.

The various technical features of the above-described embodiments may be combined in any combination, so that the description is concise, and all possible combinations of the various technical features in the above-described embodiments are described. However, as long as the combination of these technical features does not conflict, it is to be understood that the scope of the present specification is not to be taken in a limiting sense.

The above-described embodiments have only expressed several embodiments of the present application, which are described in more detail and detail, but are not therefore to be construed as limiting the scope of the present application. It should be noted that variations and modifications may be made to one of ordinary skill in the art without departing from the spirit of the present application, all of which fall within the scope of the present application. Therefore, the scope of the appended claims should be accorded the broadest interpretation so as to encompass all such modifications and similar arrangements.

## Claims

1. A surgical robot, **characterized in that**, comprising:

    a mechanical arm;
    one or more operating arms mounted on a remote end of the mechanical arm, each of the one or more operating arms comprising an end instrument, the end instrument comprising an image end instrument and/or an operating end instrument; and
    a control device, configured for:

        acquiring first target position and/or pose information of the remote end of the mechanical arm at a first coordinate system and current position and/or pose information of each of the controlled end instrument at the first coordinate system, the first coordinate system being a base coordinate of the mechanical arm;
        in a condition of the remote end of the mechanical arm reaching a target position and/or pose corresponding to the first target position and/or pose information, converting the current position and/or pose information of each of the controlled end instrument at the first coordinate system to be second target position and/or pose information of a corresponding controlled end instrument at a second coordinate system, the second coordinate system being a tool coordinate system of the mechanical arm; and
        controlling each joint assembly of the mechanical arm to linkedly move according to the first target position and/or pose information to allow the remote end of the mechanical arm to reach the target position and/or pose corresponding to the first target position and/or pose information, and controlling each joint assembly of each of the one or more operating arms to linkedly move according to a corresponding second target position and/or pose information to allow the corresponding controlled end instrument to maintain a current position and/or pose unchanged.

2. The surgical robot of claim 1, **characterized in that**, the control device is configured for:
    before the step of acquiring the current position and/or pose information of the controlled end instrument at the first coordinate system, performing:
    acquiring a selection instruction input by a user, and selecting the one or more end instruments according to the selection instruction to be the controlled end instruments.

3. The surgical robot of claim 1, **characterized in that**, after the step of converting the current position and/or pose information of each of the controlled end instrument at the first coordinate system to be the second target position and/or pose information of the corresponding controlled end instrument at the second coordinate system, the control device is configured for:

    determining if each second target position and/or pose information is effective;
    if each second target position and/or pose information is determined effective, performing the step of controlling each joint assembly of the mechanical arm to linkedly move according to the first target position and/or pose information to allow the remote end of the mechanical arm to reach the target position and/or pose corresponding to the first target position and/or pose information, and controlling each joint assembly of each of the one or more operating arms to linkedly move according to the corresponding second target position and/or pose information to allow the corresponding controlled end instrument to maintain the current position and/or pose unchanged.

4. The surgical robot of claim 3, **characterized in that**, in the step of determining if each second target position and/or pose information is effective, the control device is configured for:

    determining if each second target position and/or pose information is effective, and acquiring a corresponding determine result; and
    determining if each second target position and/or pose information is effective according to the determined result.

5. The surgical robot of claim 4, **characterized in that**,

in the step of determining if each second target position and/or pose information is effective, the control device is configured for:

analyzing the second target position and/or pose information to acquire target motion state parameters of each joint assembly in each of the one or more operating arms;

comparing the target motion state parameters of each joint assembly in each of the one or more operation arms with motion state thresholds of each joint assembly of each of the one or more operating arms; and

if the target motion state parameters of each joint assembly in each of the one or more operation arms are not greater than the motion state thresholds of each joint assembly of each of the one or more operating arms, determining that the corresponding second target position and/or pose information is effective; otherwise, determining that the corresponding second target position and/or pose information is ineffective.

6. The surgical robot of claim 5, **characterized in that**, the target motion state parameters comprise position parameters, speed parameters, and accelerated speed parameters, and the motion state thresholds comprise a position parameter threshold, a speed parameter threshold, and an accelerated speed parameter threshold.

7. The surgical robot of claim 1, **characterized in that**,

in the step of acquiring the first target position and/or pose information of the remote end of the mechanical arm at the first coordinate system, the control device is configured for acquiring configuration information of task degrees of freedom of the remote end of the mechanical arm; and acquiring the first target position and/or pose information of the remote end of the mechanical arm at the first coordinate system and associated with the corresponding task degrees of freedom according to the configuration information of the task degrees of freedom of the remote end of the mechanical arm.

8. The surgical robot of claim 1, **characterized in that**, the configuration information of the task degrees of freedom of the remote end of the mechanical arm is associated with position degrees of freedom and/or pose degrees of freedom.

9. The surgical robot of claim 1, **characterized in that**, in the step of acquiring the current position and/or pose information of each controlled end instrument at the first coordinate system, the control device is configured for:

acquiring configuration information of task degrees of freedom of each controlled end instrument; and

acquiring the current target position and/or pose information of each controlled end instrument at the first coordinate system and associated with the corresponding task degrees of freedom according to the configuration information of the task degrees of freedom of the controlled end instrument.

10. The surgical robot of claim 9, **characterized in that**, the configuration information of the task degrees of freedom of the controlled end instrument is associated with position degrees of freedom and/or pose degrees of freedom.

11. The surgical robot of claim 1, **characterized in that**, in the step of acquiring the first target position and/or pose information of the remote end of the mechanical arm at the first coordinate system, the control device is configured for:

acquiring a six-dimensional torque vector of a force applied to the mechanical arm to drag the mechanical arm to move;

analyzing the six-dimensional torque vector of the force to obtain increment position and/or pose information of the remote end of the mechanical arm at the first coordinate system;

acquiring the current position and/or pose information of the remote end of the mechanical arm at the first coordinate system; and

calculating the first target position and/or pose information according to the increment position and/or pose information and the current position and/or pose information of the remote end of the mechanical arm at the first coordinate system.

12. The surgical robot of claim 1, **characterized in that**,

in the step of acquiring the first target position and/or pose information of the remote end of the mechanical arm at the first coordinate system, the control device is configured for acquiring motion information of a motion input device;

analyzing the motion information to obtain increment position and/or pose information of the remote end of the mechanical arm at the first coordinate system;

acquiring the current position and/or pose information of the remote end of the mechanical arm at the first coordinate system; and

calculating the first target position and/or pose information according to the increment position and/or pose information and the current position and/or pose information of the remote end of the mechanical arm at the first coordinate system.

13. The surgical robot of claim 4, **characterized in that**, before the step of acquiring the first target position and/or pose information of the remote end of the mechanical arm at the first coordinate system and the current position and/or pose information of each of the controlled end instrument at the first coordinate system, the control device is configured for: acquiring description information of a structure feature of the mechanical arm and each of the one or more operating arms having the controlled end instrument and being mounted on at the remote end the mechanical arm, and generating a model image of structure features of the mechanical arm and the corresponding operating arm according to the description information.

14. The surgical robot of claim 13, **characterized in that**, after the step of determining if each second target position and/or pose information is effective, and acquiring the corresponding determine result, the control device is configured for: changing a color of the model image of the operating arm corresponding to the controlled end instrument if the second target position and/or pose information is determined to be ineffective.

15. The surgical robot of claim 1, **characterized in that**, the end instrument is the image end instrument or the operating end instrument.

16. A control method for controlling an end instrument of a surgical robot, the surgical robot comprising a mechanical arm, the mechanical arm comprising one or more operating arms mounted on a remote end thereof, the one or more operating arms each comprising an end instrument, and one or more of the end instruments being the controlled end instruments, **characterized in that**, the control method comprising:

> acquiring first target position and/or pose information of the remote end of the mechanical arm at a first coordinate system and current position and/or pose information of each of the controlled end instrument at the first coordinate system, the first coordinate system being a base coordinate of the mechanical arm;
> in a condition of the remote end of the mechanical arm reaching a target position and/or pose corresponding to the first target position and/or pose information, converting the current position and/or pose information of each of the controlled end instrument at the first coordinate system to be second target position and/or pose information of a corresponding controlled end instrument at a second coordinate system, the second coordinate system being a tool coordinate system of the mechanical arm; controlling each joint assembly of the mechanical arm to linkedly move according to the first target position and/or pose information to allow the remote end of the mechanical arm to reach the target position and/or pose corresponding to the first target position and/or pose information, and controlling each joint assembly of the one or more operating arms to linkedly move according to a corresponding second target position and/or pose information to allow the controlled end instrument to maintain a current position and/or pose unchanged.

17. The control method of claim 16, **characterized in that**, after the step of converting the current position and/or pose information of each of the controlled end instrument at the first coordinate system to a second target position and/or pose information of each of the controlled end instrument at the second coordinate system, further comprises:

> determining if each second target position and/or pose information is effective;
> if each second target position and/or pose information is effective, performing the step of controlling each joint assembly of the mechanical arm to linkedly move according to the first target position and/or pose information to allow the remote end of the mechanical arm to reach the target position and/or pose corresponding to the first target position and/or pose information, and controlling each joint assembly of each of the one or more operating arms to linkedly move according to the second target position and/or pose information to allow the corresponding controlled end instrument to maintain a current position and/or pose unchanged.

18. The control method of claim 16, **characterized in that**, in the step of acquiring the first target position and/or pose information of the remote end of the mechanical arm at the first coordinate system, comprises:

> acquiring configuration information of task degrees of freedom of the remote end of the mechanical arm; and
> acquiring the first target position and/or pose information of the remote end of the mechanical arm at the first coordinate system and associated with the corresponding task degrees of freedom according to the configuration information of the task degrees of freedom of the remote end of the mechanical arm.

**19.** The control method of claim 16, **characterized in that**, in the step of acquiring the first target position and/or pose information of the remote end of the mechanical arm at the first coordinate system, comprises:

acquiring a six-dimensional torque vector of a force applied to the mechanical arm to drag the mechanical arm to move;
analyzing the six-dimensional torque vector of the force to obtain increment position and/or pose information of the remote end of the mechanical arm at the first coordinate system;
acquiring the current position and/or pose information of the remote end of the mechanical arm at the first coordinate system; and
calculating the first target position and/or pose information according to the increment position and/or pose information and the current position and/or pose information of the remote end of the mechanical arm at the first coordinate system.

**20.** A control device of an end instrument of a surgical robot, **characterized in that**, comprising:

a storage medium, configured for storing a computer procedure;
a processor, configured for loading and carrying out the computer procedure;
wherein the computer procedure is loaded by the processor and carries out following steps of:

acquiring first target position and/or pose information of the remote end of the mechanical arm at a first coordinate system and current position and/or pose information of each of the controlled end instrument at the first coordinate system, the first coordinate system being a base coordinate of the mechanical arm;
in a condition of the remote end of the mechanical arm reaching a target position and/or pose corresponding to the first target position and/or pose information, converting the current position and/or pose information of each of the controlled end instrument at the first coordinate system to second target position and/or pose information of a corresponding controlled end instrument at a second coordinate system, the second coordinate system being a tool coordinate system of the mechanical arm;
controlling each joint assembly of the mechanical arm to linkedly move according to a first target position and/or pose information to allow the remote end of the mechanical arm to reach the target position and/or pose corresponding to the first target position and/or pose information, and controlling each joint assembly of each of the one or more operating arms to linkedly move according to a corresponding second target position and/or pose information to allow the controlled end instrument to maintain a current position and/or pose unchanged.

FIG. 1

FIG. 2

FIG. 3

Start

Acquiring first target position and/or pose information of a remote end of a mechanical arm at a first coordinate system and a current position and/or pose information of each controlled end instrument at the first coordinate system ⌐S1

Under a condition of the remote end of the mechanical arm reaching a target position and/or pose corresponding to a first target position and/or pose information, converting the current position and/or pose information of each the controlled end instrument at the first coordinate system to a second target position and/or pose information of each the controlled end instrument at a second coordinate system ⌐S2

If each the second target position and/or pose information is effective ⌐S4

NO

YES

Controlling joint assemblies of the mechanical arm to linkably move according to the first target position and/or pose information, and controlling joint assemblies of the corresponding operating arm to linkably move according to the second target position and/or pose information ⌐S3

End — S5

FIG. 4

FIG. 5

| Determining if the second target position and/or pose information of each controlled operating end instrument is effective | ~S41 |
|---|---|

| Determining if the second target position and/or pose information is effective according to the above determined result | ~S42 |
|---|---|

FIG. 6

| Analyzing the second target position and/or pose information to acquire target motion state parameters of each joint assembly of a corresponding operating arm | ~S411 |
|---|---|

| Comparing the target motion state parameters of each joint assembly in each of the operation arms to a motion state thresholds of each joint assembly of the operating arm | ~S412 |
|---|---|

FIG. 7

Acquiring the doctor's operation information of the mechanical arm, and analyzing and mapping the operation information to an incremental position and/or pose information of the remote end of the mechanical arm in the first coordinate system ⌐S11

Acquiring a current position and/or pose information of the remote end of the mechanical arm ⌐S12

According to the incremental position and/or pose information and the current position and/or pose information of the remote end of the mechanical arm at the first coordinate system, calculating its target position and/or pose information ⌐S13

FIG. 8

FIG. 9

FIG. 10

Calculating the target position information of each joint assembly of an arm body mechanism according to target position and/or pose information of the arm body mechanism ⌐S31

↓

According to the target position information of each joint assembly, controlling the joint assemblies of the arm body mechanism to linkably move to allow a remote end of the arm body mechanism to move to a target position and/or pose ⌐S32

FIG. 11

Start

S100

If a startup command for starting an activecontrol is acquired

NO

S1

Acquiring a first target position and/or pose information of the remote end of the mechanical arm at the first coordinate system and a current position and/or pose information of a controlled end instrument at the first coordinate system

S2

Under a condition of the remote end of the mechanical arm reaching a target position and/or pose corresponding to a first target position and/or pose information, converting the current position and/or pose information of each the controlled end instrument at the first coordinate system to a second target position and/or pose information of each the controlled end instrument at a second coordinate system

NO

S3

Controlling joint assemblies of the mechanical arm to linkably move according to the first target position and/or pose information, and controlling the joint assemblies of the corresponding operating arm to linkably move according to the second target position and/or pose information

S101

If end command is acquired

YES

End

FIG. 12

Acquiring the first position and/or pose information of the motion input device in a previous time $\quad$ S111

Acquiring the second position and/or pose information of the input device at a next time $\quad$ S112

According to the first position and/or pose information and the second position and/or pose information of the motion input device, calculating the incremental position and/or pose information of the motion input device in the fixed coordinate system $\quad$ S113

Mapping the incremental position and/or pose information of the motion input device in the fixed coordinate system to the incremental position information of the remote end of the mechanical arm at the first coordinate system $\quad$ S114

FIG. 13

second
position
and/or pose

first
position
and/or
pose

11

12

FIG. 14

| Acquiring a first position and/or pose information of each of the two motion input devices at a previous time | S111' |

| Acquiring a second position and/or pose information of each of the two motion input devices at a next time | S112' |

| Combining a proportional coefficient and the respective first position and/or pose information, second position and/or pose information of the two motion input devices, and calculating the incremental position and/or pose information of the two motion input devices in the fixed coordinate system | S113' |

| Mapping the incremental position and/or pose information of the two motion input devices in the fixed coordinate system to the incremental position and/or pose information of the remote end of the mechanical arm at the first coordinate system | S114' |

FIG. 15

FIG. 16

| |
|---|
| Acquiring a first position information of each of the two motion input devices at a previous time |

S231

| |
|---|
| Acquiring the second position information of each of the two motion input devices at a next time |

S232

| |
|---|
| Combining the proportional coefficient and the respective first position information, the second position information of the two motion input devices, calculating the horizontal moving increment information, vertically moving increment information and rotation increment information of the two motion input devices |

S233

FIG. 17

Establishing a first position vector between the two motion input devices at a previous time ⌐S2331

↓

Establishing a second position vector between the two motion input devices at a next time is established ⌐S2332

↓

Combining the proportional coefficient to calculate an angle between the first position vector and the second position vector, thus to acquire the rotation increment information of the two motion devices at the fixed coordinate system ⌐S2333

↓

Mapping the horizontal increment movement, the vertical movement increment information, and the rotation incremental information of the two motion input devices in the fixed coordinate system one-to-one to the yaw angle increment information, the pitch angle incremental information and the rolling angle increment information of the remote end of the mechanical arm ⌐S2334

FIG. 18

a first
position
vector

a second
position
vector

FIG. 19

FIG. 20

## INTERNATIONAL SEARCH REPORT

| | International application No. |
|---|---|
| | **PCT/CN2020/114115** |

| **A.** | **CLASSIFICATION OF SUBJECT MATTER** |
|---|---|
| | A61B 34/37(2016.01)i; A61B 34/30(2016.01)i |

According to International Patent Classification (IPC) or to both national classification and IPC

| **B.** | **FIELDS SEARCHED** |
|---|---|

Minimum documentation searched (classification system followed by classification symbols)

    A61B34/-

Documentation searched other than minimum documentation to the extent that such documents are included in the fields searched

Electronic data base consulted during the international search (name of data base and, where practicable, search terms used)

    CNKI, CNPAT, WPI, EPODOC: 深圳市精锋医疗, 高元倩, 叶元强, 机械臂, 操作臂, 机器人, 远端, 位置, 姿态, 位姿, 姿势, 目标, 保持, 不变, 器械, 工具, 基坐标系, 工具坐标系, 器械坐标系, 不动点, 运动中心, 约束中心, mechanical arm, robotic arm, operating arm, robot, surgical, position, posture, target, aiming, maintain, constant, instrument, tool, base coordinate system, tool coordinate system, instrument coordinate system, remote center, motion

| **C.** | **DOCUMENTS CONSIDERED TO BE RELEVANT** |
|---|---|

| Category* | Citation of document, with indication, where appropriate, of the relevant passages | Relevant to claim No. |
|---|---|---|
| PX | CN 110559083 A (EDGE MEDICAL ROBOTICS CO.,LTD.) 13 December 2019 (2019-12-13)<br>    description paragraphs [0048]-[0191], claims 1-10, figures 1-20 | 1-20 |
| A | CN 109965982 A (EDGE MEDICAL ROBOTICS CO.,LTD.) 05 July 2019 (2019-07-05)<br>    description paragraphs [0028]-[0040], [0095]-[0101], figures 1-3 | 1-20 |
| A | CN 105014677 A (XI'AN JIAOTONG UNIVERSITY) 04 November 2015 (2015-11-04)<br>    entire document | 1-20 |
| A | CN 109974584 A (SHANDONG UNIVERSITY) 05 July 2019 (2019-07-05)<br>    entire document | 1-20 |
| A | CN 108210070 A (MICROPORT (SHANGHAI) MEDICAL ROBOT CO., LTD.) 29 June 2018 (2018-06-29)<br>    entire document | 1-20 |
| A | CN 110169825 A (HARBIN INSTITUTE OF TECHNOLOGY) 27 August 2019 (2019-08-27)<br>    entire document | 1-20 |

☑ Further documents are listed in the continuation of Box C.      ☑ See patent family annex.

| | |
|---|---|
| *     Special categories of cited documents:<br>"A"   document defining the general state of the art which is not considered to be of particular relevance<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
|---|---|
| **18 November 2020** | **27 November 2020** |

| Name and mailing address of the ISA/CN | Authorized officer |
|---|---|
| **China National Intellectual Property Administration (ISA/CN)**<br>**No. 6, Xitucheng Road, Jimenqiao Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

| International application No. |
| --- |
| **PCT/CN2020/114115** |

**C.    DOCUMENTS CONSIDERED TO BE RELEVANT**

| Category* | Citation of document, with indication, where appropriate, of the relevant passages | Relevant to claim No. |
| --- | --- | --- |
| A | US 2016361125 A1 (THE JOHNS HOPKINS UNIVERSITY) 15 December 2016 (2016-12-15) entire document | 1-20 |

Form PCT/ISA/210 (second sheet) (January 2015)

### INTERNATIONAL SEARCH REPORT
**Information on patent family members**

International application No.

**PCT/CN2020/114115**

| Patent document cited in search report | | | Publication date (day/month/year) | Patent family member(s) | | | Publication date (day/month/year) |
|---|---|---|---|---|---|---|---|
| CN | 110559083 | A | 13 December 2019 | CN | 110559083 | B | 25 August 2020 |
| CN | 109965982 | A | 05 July 2019 | CN | 109965980 | A | 05 July 2019 |
| | | | | CN | 109223183 | A | 18 January 2019 |
| | | | | CN | 109965981 | A | 05 July 2019 |
| | | | | CN | 109965982 | B | 09 October 2020 |
| | | | | CN | 109965981 | B | 22 September 2020 |
| | | | | CN | 109965980 | B | 09 October 2020 |
| CN | 105014677 | A | 04 November 2015 | CN | 105014677 | B | 20 July 2016 |
| CN | 109974584 | A | 05 July 2019 | CN | 109974584 | B | 20 March 2020 |
| CN | 108210070 | A | 29 June 2018 | CN | 108210070 | B | 10 April 2020 |
| CN | 110169825 | A | 27 August 2019 | | None | | |
| US | 2016361125 | A1 | 15 December 2016 | US | 10166080 | B2 | 01 January 2019 |
| | | | | WO | 2016201303 | A1 | 15 December 2016 |

Form PCT/ISA/210 (patent family annex) (January 2015)

**REFERENCES CITED IN THE DESCRIPTION**

**Patent documents cited in the description**

- CN 201910854118